# EUROPÄISCHE PATENTSCHRIFT

(11) **EP 1 784 119 B1**
(45) Veröffentlichungstag und Bekanntmachung des Hinweises auf die Patenterteilung: **10.03.2010**
(21) Anmeldenummer: 05776641.2
(22) Anmeldetag: 10.08.2005
(51) Int. Cl.: A61B 1/00

(54) **STECKEREINHEIT FÜR ENDOSKOPE**
CONNECTOR UNIT FOR ENDOSCOPES
UNITE DE CONNEXION POUR ENDOSCOPES

(30) Priorität: 19.08.2004 DE 202004012991 U
(43) Veröffentlichungstag der Anmeldung: 16.05.2007
(73) Patentinhaber: Storz Endoskop Produktions GmbH, 78532 Tuttlingen (DE)
(72) Erfinder: KRATTIGER, Beat, CH-8222 Beringen (CH); KLUMPP, Martin, 78532 Tuttlingen (DE); KUSTER, Manfred, CH-9443 Widnau (CH)
(74) Vertreter: Stamer, Harald
(86) Internationale Anmeldenummer: PCT/EP2005/008664
(87) Internationale Veröffentlichungsnummer: WO 2006/018200

(56) Entgegenhaltungen:
- EP-A- 0 726 059
- US-A1- 2002 040 181
- US-A1- 2003 220 545

## Beschreibung

Die Erfindung betrifft eine Steckereinheit mit Integralkabelverbindung zum Bedienteil eines Endoskops und Steckanschlüssen für Lampen-, Mechanik- und/oder Elektronik-Module.

Endoskope, insbesondere für industrielle Meß- und Inspektionsaufgaben, enthalten üblicherweise eine Mehrzahl von Peripheriegeräten für die Stromversorgung, Beleuchtung, Kamerasteuerung, Bildauswertung, Monitore usw. Um die Handhabung des Endoskops mit seinem Bedienteil nicht unnötig zu behindern, ist eine Steckereinheit vorgesehen, von der aus über eine Integralkabelverbindung die optische und elektronische Versorgung, sowie der Datenaustausch zum Endoskop geleitet wird. Die Anzahl der Steckanschlüsse entspricht der Anzahl der möglichen Peripheriegeräte für das jeweilige Endoskop, so daß die Steckereinheit im wesentlichen einen einfachen Austausch unterschiedlicher Peripheriegeräte desselben Typs ermöglicht. Die Steckereinheit ist so gestaltet, daß die mechanische Beanspruchung der Integralkabelverbindung beim Anschluß der Peripheriegeräte möglichst gering ist.

Der Erfindung liegt die Aufgabe zugrunde, die Steckereinheit so weiterzubilden, daß neu entwickelte oder für das jeweilige Endoskop zunächst nicht vorgesehene Zusatzmodule ohne Umbau oder Zerlegung der übrigen Steckanschlüsse nachrüstbar sind. Das Modul sollte dabei so mit der Steckereinheit verbindbar sein, daß die statischmechanische Belastung der Integralkabelverbindung nicht nachteilig beeinflußt wird.

Diese Aufgabe wird dadurch gelöst, daß in die Steckereinheit über die Integralkabelverbindung und das Bedienteil ein bis zum distalen Ende des Endoskops verlaufendes Leerrohr eingefügt ist, dessen proximales Ende in der Steckereinheit frei beweglich so angeordnet ist, daß es aus einer verschließbaren Bohrung in der Wandung der Steckereinheit herausführbar ist. In die offene Bohrung kann eine Hohlachse zur Aufnahme des Leerrohres einsetzbar sein. Auf die Hohlachse kann ein Zusatzmodul so aufgesteckt sein, daß das funktionsbestimmende Teil des Zusatzmoduls der proximalen Öffnung des Leerrohres zugeordnet ist. Das Gehäuse des Zusatzmoduls ist dabei zweckmäßigerweise in einer die Steckereinheit umgreifenden Formgebung ausgebildet, deren Schwerpunkt auf der Achse der Integralkabelverbindung liegt. In das Leerrohr kann eine Singlemode-Laserfaser eingefügt sein, der distalseitig eine Kollimationsoptik zugeordnet ist und der proximalseitig eine im Gehäuse angeordnete Laserdiode vorgeschaltet ist.

Ein Ausführungsbeispiel der Erfindung ist in der Zeichnung schematisch dargestellt. Dabei zeigen:
- Fig.1: eine Gesamtansicht mit Steckereinheit, Integralkabelverbindung und Bedienteil eines Endoskops,
- Fig.2: eine perspektivische Aufsicht auf die Steckereinheit mit Bedienseite eines Zusatzmoduls und
- Fig.3: einen Querschnitt durch die Steckereinheit und das Zusatzmodul.

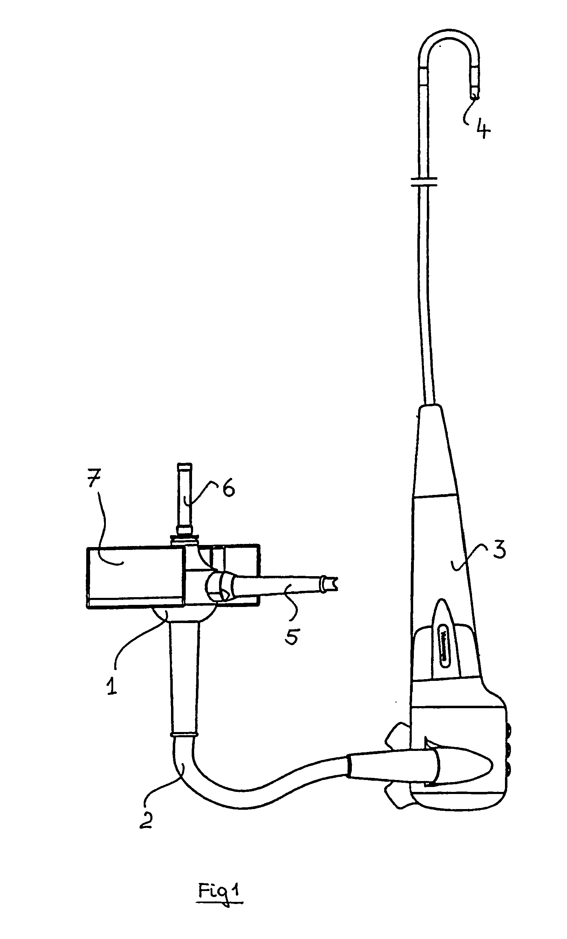

Eine in Fig.1 dargestellte Steckereinheit 1 ist über eine Integralkabelverbindung 2 mit dem Bedienteil 3 eines Endoskops verbunden. Bei dem Endoskop handelt es sich beispielsweise um ein Video-Meßendoskop, dessen distales Ende 4 über Bowdenzüge bewegbar ist. Bei dem Meßendoskop kann es sich um ein Vielpunkt- oder ein Zweipunkt-Meßsystem handeln, das über die Steckereinheit 1 versorgt wird.

Die Steckereinheit 1 weist zwei Steckanschlüsse 5, 6 auf. Der Steckanschluß 5 enthält z.B. die Leitungen zur Kamerasteuerung und Bildauswertung und auf den Steckanschluß 6 kann z.B. eine Beleuchtungseinheit aufgesetzt werden. Weitere Steckanschlüsse z.B. für die Stromversorgung sind hier nicht weiter dargestellt.

Auf die Steckereinheit 1 ist ein Gehäuse 7 für ein Zusatzmodul aufgesteckt. Das Gehäuse 7 ist in seiner Formgebung so ausgestaltet, daß es die Steckereinheit 1 von der Seite her umgreift, wobei insbesondere der Steckanschluß 5 frei bleibt. Das Gehäuse 7 ist dabei so konstruiert, daß sein Schwerpunkt auf der durch die Steckereinheit 1 verlaufende Längsachse der Integralkabelverbindung 2 liegt. Bei einem Zug an der Steckereinheit 1 aufgrund der Bedienung und Bewegung des Endoskops kann die Integralkabelverbindung 2 daher die Kräfte aufnehmen, ohne daß zusätzliche Querkräfte entstehen. Das Gehäuse 7 des Zusatzmoduls wird somit zu einem Bestandteil der Steckereinheit 1.

Das Gehäuse 7 des Zusatzmoduls kann für unterschiedliche Anwendungen ausgelegt sein, die über ein von der Steckereinheit 1 ausgehendes und über die Integralkabelverbindung 2 und das Bedienteil 3 zum distalen Ende 4 führendes Leerrohr an einem zu untersuchenden Objektbereich ausgeführt werden können. Das proximale Ende des Leerrohres ist in der Steckereinheit 1 zunächst frei beweglich so angeordnet, daß es bei Bedarf aus einer durch einen Stopfen verschlossenen Bohrung in der Wandung der Steckereinheit 1 herausgeführt werden kann. Zur Vorbereitung für das Ansetzen des Zusatzmoduls wird die Bohrung geöffnet und eine Hohlachse in die Wandung eingesetzt, in die dann das proximale Ende des Leerrohres hineingezogen wird. Je nach Ausstattung des Zusatzmoduls wird dessen funktionsbestimmendes Teil in dem Gehäuse 7 so angeordnet, daß es mit der Öffnung des Leerrohres zusammenwirken kann. Die Hohlachse dient dabei zum Ausrichten und Halt des Gehäuses 7 auf der Steckereinheit 1. Mit Hilfe eines zusätzlichen Paßstiftes kann das Gehäuse 7 auf der Steckereinheit 1 gegen eine Verdrehung gesichert werden.

Ein erstes Zusatzmodul kann zur Einstrahlung von Laserlicht auf das Objekt vorgesehen sein. Dazu wird in das Leerrohr eine Singlemode-Laserfaser eingeschoben, der distalseitig eine Kollimationsoptik zugeordnet wird. Proximalseitig enthält das Gehäuse 7 als funktionsbestimmendes Teil eine Laserdiode, deren Strahlung in die Singlemode-Laserfaser eingekoppelt wird. Zur Energieversorgung der Laserdiode kann an dem Gehäuse 7 eine geeignete Steckverbindung zum Anschluß an eine Versorgungseinheit vorgesehen sein. Die Nachrüstung des Systems wird vorteilhafterweise beim Hersteller vorgenommen, um die gegenseitige Justierung der Funktionselemente sicherzustellen. Eine Zerlegung der bestehenden Anschlüsse ist erkennbar nicht notwending.

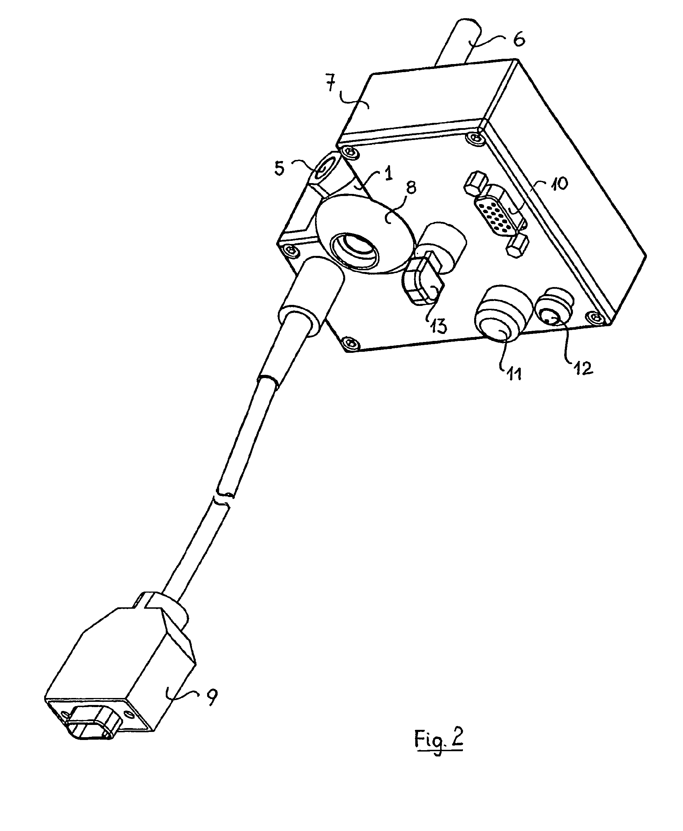

Fig.2 zeigt ein solches Zusatzmodul in perspektivischer Aufsicht auf seine Bedienseite. Von der Steckereinheit 1 ist der kalottenförmige Ansatz 8 dargestellt, in den die Integralkabelverbindung 2 eingesetzt wird. An der Steckereinheit 1 ist seitlich der Ansatz für den Steckanschluß 5 zur Kamerasteuerung zu sehen und nach oben weisend der Steckanschluß 6 für eine Beleuchtungseinheit. Das Gehäuse 7 des Zusatzmoduls umschließt die Steckereinheit 1 von der Seite her, wobei der Bereich des Steckanschlusses 5 frei bleibt. Das Gehäuse 7 ist zusätzlich mit einem Steckanschluß 9 zur Energieversorgung versehen. Außerdem ist ein Steckersockel 10 zum Anschluß zusätzlicher externer Geräte vorgesehen. Über einen Potentiometer-Drehknopf 11 kann die Beleuchtungsintensität geregelt werden. Eine Anzeigelampe 12 dient zur Betriebsanzeige. Über einen Schlüsselschalter 13 ist eine zentrale Inbetriebnahme des Zusatzmoduls möglich.

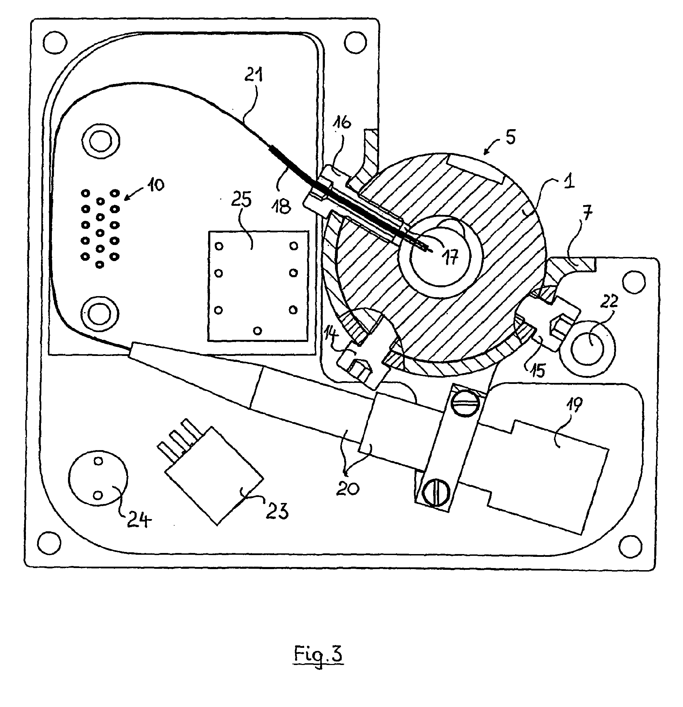

Die in Fig.3 dargestellte Schnittdarstellung entspricht einer Aufsicht auf die Rückseite des Zusatzmoduls. Auf die Steckereinheit 1 ist das Gehäuse 7 formschlüssig aufgeschoben und wird über als Schrauben ausgebildete Paßstifte 14, 15 mit der Steckereinheit 1 drehsicher verbunden. Die Orientierung von Steckereinheit 1 und Gehäuse 7 zueinander ist so gewählt, daß eine in die Wandung des Gehäuses 7 eingesetzte Hohlachse 16 mit einer Bohrung 17 in der Steckereinheit 1 fluchtet. Durch die Bohrung 17 und die Hohlachse 16 hindurch ist ein Leerrohr 18 aus der hier nicht weiter dargestellten Intrgralkabelverbindung in das Gehäuse 7 hineingeführt.

Innerhalb des Gehäuses 7 ist in einer Fassung 19 eine Laserdiode angeordnet. Die Fassung 19 enthält eine optische Steckverbindung 20 zum Anschluß einer Laserfaser 21, die in das Leerrohr 18 eingeführt ist.

Als Laserlichtquelle kann insbesondere eine im sichtbaren Wellenlängenbereich strahlende Laserdiode vorgesehen sein. Vorzugsweise sollte die Laserdiode bei 639 nm ±3 nm arbeiten. Zur Ankopplung der Singlemode-Laserfaser an die Laserdiode dient die lösbare Steckverbindung 20. Durch die Lösbarkeit der Verbindung werden Reparaturen erleichtert und die Zuordnung zur Eintrittsfläche der Singlemode-Laserfaser sichergestellt.

Die verwendete Laserdiode und/oder die Laserdioden-Ansteuerung sollten so ausgelegt sein, daß Speckles im Strahlungsfeld reduziert sind. Dazu können Speckles reduzierende Mittel zwischen Laserdiode und Eintrittsfläche der Singlemode-Laserfaser angeordnet werden. Die Erkennbarkeit des Meßmusters auf dem Objekt wird durch Unterdrückung von Speckles deutlich verbessert.

In der Schnittdarstelltung erkennbar sind außerdem die Durchtrittsöffnung 22 für die mit dem Steckanschluß 9 verbundenen Energieversorgungsleitungen, ein mit dem Drehknopf 11 verstellbares Potentiometer 23, ein Lampensockel 24 für die Anzeigelampe 12 und eine Schaltplatine 25 für den Schlüsselschalter 13.

Als weitere Ausstattungen für Zusatzmodule können z.B. Schleifeinrichtungen, Sensoren , Manipulationseinrichtungen, andere Zusatzbeleuchtungen, Sprüh-, Disponier- oder Dosiereinrichtungen vorgesehen sein, deren Anwendung durch über das Leerrohr geführte Betätigungselemente und Steuerungsmittel am Gehäuse 7 ausgelöst werden kann.

### Bezucgszeichenliste

- 1: Steckereinheit
- 2: Integralkabelverbindung
- 3: Bedienteil
- 4: distales Ende
- 5: Steckanschluß für Kamera
- 6: Steckanschluß für Beleuchtung
- 7: Gehäuse
- 8: kalottenförmiger Ansatz an der Steckereinheit
- 9: Steckanschluß für Einergieversorgung
- 10: Steckersockel
- 11: Potentiometer-Drehknopf
- 12: Anzeigelampe
- 13: Schlüsselschalter
- 14/15: Paßstifte
- 16: Hohlachse
- 17: Bohrung in Steckereinheit
- 18: Leerrohr
- 19: Fassung
- 20: optische Steckverbindung
- 21: Laserfaser
- 22: Durchtrittsöffnung für Energieversorgungsleitungen
- 23: Potentiometer
- 24: Lampensockel
- 25: Schaltplatine

## Patentansprüche

1. Steckereinheit mit Integralkabelverbindung zum Bedienteil eines Endoskops und Steckanschlüssen für Lampen-, Mechanik- und/oder Elektronik-Module in Kombination mit einem Endoskop, **dadurch gekennzeichnet, daß** in die Steckereinheit (1) über die Integralkabelverbindung (2) und das Bedienteil (3) ein bis zum distalen Ende (4) des Endoskops verlaufendes Leerrohr eingefügt ist, dessen proximales Ende in der Steckereinheit (1) frei beweglich so angeordnet ist, daß es aus einer verschließbaren Bohrung (17) in der Wandung der Steckereinheit (1) herausführbar ist.

2. Steckereinheit nach Anspruch 1, **dadurch gekennzeichnet, daß** in die offene Bohrung (17) eine Hohlachse (16) zur Aufnahme des Leerrohres (18) einsetzbar ist.

3. Steckereinheit nach Anspruch 2, **dadurch gekennzeichnet, daß** die Hohlachse (16) auf ein Zusatzmodul so aufgesteckt ist, daß das funktionsbestimmende Teil des Zusatzmoduls der proximalen Öffnung des Leerrohres (18) zugeordnet ist.

4. Steckereinheit nach Anspruch 3, **dadurch gekennzeichnet, daß** das Gehäuse (7) des Zusatzmoduls in einer die Steckereinheit (1) umgreifenden Formgebung ausgebildet, deren Schwerpunkt auf der Achse der Integralkabelverbindung (2) liegt.

5. Steckereinheit nach Anspruch 3, **dadurch gekennzeichnet, daß** in das Leerrohr (18) eine Singlemode-Laserfaser eingefügt ist, der distalseitig eine Kollimationsoptik zugeordnet ist und der proximalseitig eine im Gehäuse (7) in einer Fassung (19) angeordnete Laserdiode vorgeschaltet ist.

## Claims

1. Plug unit with an integral cable connection to the operating part of an endoscope and plug-type connections for lamp, mechanical and/or electronics modules in combination with an endoscope, **characterized in that** an empty conduit which runs up to the distal end (4) of the endoscope is inserted into the plug unit (1) via the integral cable connection (2) and the operating part (3), the proximal end of said empty conduit being arranged in freely movable fashion in the plug unit (1) in such a way that it can be guided out of a closable bore (17) in the wall of the plug unit (1).

2. Plug unit according to Claim 1, **characterized in that** a hollow shaft (16) for accommodating the empty conduit (18) can be inserted into the open bore (17).

3. Plug unit according to Claim 2, **characterized in that** the hollow shaft (16) is plugged onto an additional module in such a way that the function-determining part of the additional module is associated with the proximal opening of the empty conduit (18).

4. Plug unit according to Claim 3, **characterized in that** the housing (7) of the additional module is designed to have a shape which surrounds the plug unit (1) and whose centre of gravity is on the axis of the integral cable connection (2).

5. Plug unit according to Claim 3, **characterized in that** a single-mode laser fibre is inserted into the empty conduit (18), with a collimating optic being associated with said single-mode laser fibre on the distal side and, on the proximal side, a laser diode which is arranged in the housing (7) in a holder (19) being connected upstream of said single-mode laser fibre.

## Revendications

1. Unité de connexion avec liaison par câble intégrée vers la partie commande d'un endoscope et raccords enfichables pour des modules de lampes, mécaniques et/ou électroniques en combinaison avec un endoscope, **caractérisée en ce qu'**un tube vide qui s'étend jusqu'à l'extrémité distale (4) de l'endoscope est inséré dans l'unité de connexion (1) sur la liaison par câble intégrée (2) et la partie commande (3), dont l'extrémité proximale est disposée de manière à pouvoir bouger librement dans l'unité de connexion (1) de telle sorte qu'elle peut être amenée à l'extérieur depuis un orifice (17) pouvant être fermé dans la paroi de l'unité de connexion (1).

2. Unité de connexion selon la revendication 1, **caractérisée en ce qu'**un axe creux (16) destiné à accueillir le tube vide (18) peut être inséré dans l'orifice ouvert (17).

3. Unité de connexion selon la revendication 2, **caractérisée en ce que** l'axe creux (16) est emmanché sur un module supplémentaire de telle sorte que la partie du module supplémentaire qui détermine la fonction est associée à l'ouverture proximale du tube vide (18).

4. Unité de connexion selon la revendication 3, **caractérisée en ce que** le boîtier (7) du module supplémentaire est réalisé dans une forme qui enveloppe l'unité de connexion (1) dont le centre de gravité se trouve sur l'axe de la liaison par câble intégrée (2).

5. Unité de connexion selon la revendication 3, **caractérisée en ce qu'**une fibre à laser monomodale est introduite dans le tube vide (18), à l'extrémité distale de laquelle est associée une optique de collimation et dont l'extrémité proximale est précédée d'une diode laser disposée dans une monture (19) dans le boîtier (7).
